# EUROPÄISCHE PATENTSCHRIFT

(11) **EP 1 616 889 B1**
(45) Veröffentlichungstag und Bekanntmachung des Hinweises auf die Patenterteilung: **11.04.2007**
(21) Anmeldenummer: 05014406.2
(22) Anmeldetag: 02.07.2005
(51) Int. Cl.: C08G 18/38, C09D 175/02, C07C 229/24

(54) **Wasserlösliche Aspartate**
Water-soluble aspartates
Aspartates hydrosolubles

(30) Priorität: 15.07.2004 DE 102004034271
(43) Veröffentlichungstag der Anmeldung: 18.01.2006
(73) Patentinhaber: Bayer MaterialScience AG, 51368 Leverkusen (DE)
(72) Erfinder: Köhler, Burkhard, Dr., 51373 Leverkusen (DE); Niesten, Meike, Dr., 51061 Köln (DE); Simon, Joachim, Dr., 40589 Düsseldorf (DE); Wamprecht, Christian, Dr., 41472 Neuss (DE)

(56) Entgegenhaltungen:
- EP-A- 0 403 921
- EP-A- 0 818 492
- EP-A- 0 849 301

## Beschreibung

Die vorliegende Erfindung betrifft neue wasserlösliche Polyasparaginsäureester sowie deren Verwendung in wässrigen Beschichtungsmitteln.

Zweikomponenten-Beschichtungsmittel, die als Bindemittel eine Polyisocyanatkomponente in Kombination mit einer gegenüber Isocyanatgruppen reaktionsfähigen Reaktivkomponente, insbesondere einer Polyhydroxylkomponente, enthalten, sind seit langem bekannt. Sie eignen sich zur Herstellung von hochwertigen Überzügen, die hart, elastisch, abrieb- und lösungsmittelbeständig eingestellt werden können.

Aufgrund der Tatsache, dass primäre Amine mit Polyisocyanaten eine spontane und fast unkontrollierbare Reaktion eingehen, sind diese als Reaktionspartner für Polyisocyanate in Zweikomponenten Beschichtungen ungeeignet.

In EP-A 0 403 921 werden niedrigviskose Polyasparaginsäureester beschrieben, welche sekundäre Aminogruppen aufweisen und daher eine verglichen mit primären Aminen gemäßigtere Reaktivität gegenüber NCO-Gruppen aufweisen. Solche Polyasparaginsäureester eignen sich besonders zur Herstellung lösemittelarmer oder -freier Beschichtungsmittel, welche eine rasche Aushärtung zeigen.

Solche Polyasparaginsäureester sind allerdings nicht wasserlöslich oder wasserdispergierbar, so dass sie sich nicht für den Einsatz in wässrigen Bindemitteln eignen.

Wegen der immer strenger werdenden Gesetzgebung bezüglich erlaubter Anteile flüchtiger organischer Komponenten beispielsweise in Beschichtungsmitteln sind wässrige Systeme zunehmend gefragter. Wässrige Zweikomponenten-Beschichtungsmittel sind seit Jahren bekannt und beispielsweise in EP-A 0 358 979 beschrieben.

In EP-B 0 818 492 werden wässrige sekundäre Polyamine beschrieben, welche durch Michael Addition von Polyaminen an Maleinsäureester-Einheiten aufweisende Oligoester erhalten werden. Ein Nachteil solcher Produkte ist, dass nach Reaktion mit dem Polyisocyanat Hydantoinbildung stattfinden kann, wodurch die Oligesterketten gespalten werden. Dies wiederum, führt zu einem Verlust der mechanischen Eigenschaften der Beschichtung.

In EP-A 0 849 301 werden wässrige Bindemittelgemische auf Basis von Polyasparaginsäureestern und wässrigen Polyisocyanaten beschrieben. Nachteilig ist jedoch, dass diese Polyasparaginsäureester nicht wasserdispergierbar oder wasserlöslich sind, so dass sie nicht als lagerstabile wässrige Formulierungen bereitgestellt werden können. Die Herstellung der Beschichtungen muss daher so erfolgen, dass das wässrige Polyisocyanat zum hydrophoben Polyasparaginsäureester gegeben wird, dann mit Wasser vermischt und sofort appliziert werden muss.

Aus Gründen der besseren Verarbeitbarkeit wäre es jedoch wünschenswert, in solchen Systemen wasserlösliche oder wasserdispergierbare Aspartate bzw. deren wässrige Formulierungen einsetzen zu können.

Aufgabe dieser Erfindung war von daher die Bereitstellung von wasserlöslichen Polyasparaginsäureestern für die Herstellung von wässrigen 2K-Beschichtungsmitteln zur Herstellung von Polyharnstoff- und/oder Polyharnstoff-Polyurethan-Beschichtungen.

Es wurde nun gefunden, dass sich wasserlösliche Polyasparaginsäureester herstellen lassen, in dem Amine mit Maleinsäureestern umgesetzt werden, die ein oder mehrere Ethylenoxid Einheiten enthalten.

Gegenstand der Erfindung sind daher hydrophile Polyasparaginsäureester der allgemeinen Formel (I), 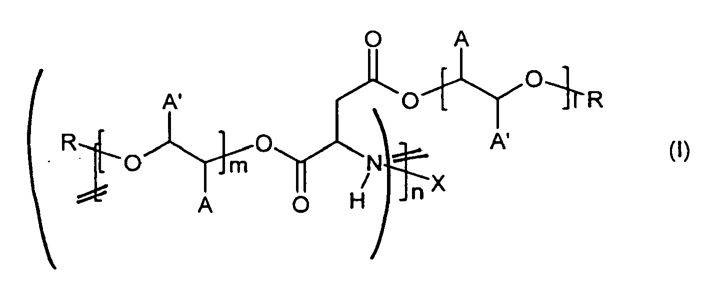 wobei
- R: ein C₁-C₄-Alkylrest ist,
- A, A': unabhängig voneinander Wasserstoff oder Methyl sind,
- X: ein n-valenter aliphatischer, araliphatischer oder cycloaliphatischer Rest mit 1 bis 20 C-Atomen ist, der gegebenenfalls Etherbrücken enthält,
- l, m n: atürliche Zahlen von 0 bis 10 sind deren Summe 1 + m = 1 bis 20 beträgt sowie
- n: eine natürliche Zahl von 1 bis 3 ist.

Bevorzugt sind A und A' Wasserstoff.

Bevorzugt ist X ein n-valenter aliphatischer oder cycloaliphatischer Rest mit 1 bis 10 C-Atomen, der gegebenenfalls Etherbrücken enthält.

Bevorzugt sind 1 und m unabhängig voneinander natürliche Zahlen von 1 bis 3.

Bevorzugt beträgt die Summe aus 1 + m = 2 bis 6.

Typischerweise haben die erfindungsgemäßen Polyasparaginsäureester zahlenmittlere Molekulargewichte von 300 g/mol bis 1000 g/mol, bevorzugt 400 g/mol bis 750 g/mol.

Typischerweise haben die erfindungsgemäßen Polyasparaginsäureester Viskositäten (gemessen mit einem Rotationsviskosimeter) bei 23°C von 50 bis 5000 mPa·s, bevorzugt 50 bis 2500 mPa·s.

Besonders vorteilhaft bei den erfindungsgemäßen Polyasparaginsäureestern ist, dass sie bereits ohne Neutralisation (ganz oder teilweise Protonierung der vorhandenen Aminofunktionen) in Wasser löslich sind.

Ferner ist Gegenstand der Erfindung ein Verfahren zur Herstellung der erfindungsgemäßen Polyasparaginsäureester, bei dem Maleinsäureester der allgemeinen Formel (II), 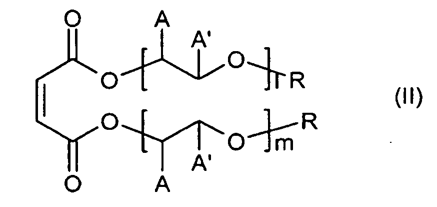 mit einem n-valenten Amin der Formel (III), 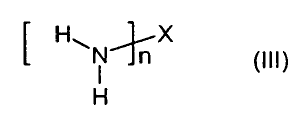 bei Temperaturen von 0. bis 100°C, vorzugsweise von 15 bis 50°C bevorzugt lösemittelfrei umgesetzt werden.

Die Bedeutung von n, X, R, A, A', 1 und m entspricht den vorstehend für Formel (I) angegebenen Definitionen.

Als Amine der Formel (II) werden besonders bevorzugt Ethylendiamin, 1,2-Diaminopropan, 1,4-Diaminobutan, 1,6-Diaminohexan, 2,5-Diamino-2,5-dimethylhexan, 2,2,4- und/oder 2,4,4-Trimethyl-1,6-diaminohexan, 1,11-Diaminoundecan, 1,12-Diaminododecan, 4-Aminoethyl-1,8-Diaminooctan, 1-Amino-3,3,5-trimethyl-5-aminomethyl-cyclohexan, 2,4-und/oder 2,6-Hexahydrotoluylendiamin, 2,4'-und/oder 4,4'-Diamino-dicyclohexylmethan, 3,3'-Dimethyl-4,4'-diamino-dicyclohexylmethan, 2,4,4'-Triamino-5-methyl-dicyclohexylmethan und Polyetherpolyaminen mit aliphatisch gebundenen primären Aminogruppen mit einem zahlenmittleren Molekulargewicht Mₙ von 148 bis 6000 g/mol eingesetzt.

Die Maleinsäureester der Formel (II) sind durch vollständige oder teilweise Umesterung von Maleinsäureestern mit monohydroxyfunktionellen Ethern zugänglich.

Bevorzugt werden als Maleinsäureester Maleinsäuredimethylester, Maleinsäurediethylester, Maleinsäuredipropylester, Maleinsäurediisopropylester, Maleinsäuredibutylester, Maleinsäureditert-butylester, Maleinsäuredi-sec-butylester oder Maleinsäurediisobutylester eingesetzt. Besonders bevorzugt sind Maleinsäuredimethylester oder Maleinsäurediethylester, ganz besonders bevorzugt Maleinsäuredimethylester.

Die monohydroxyfunktionellen Ether entsprechen der Formel (IV), 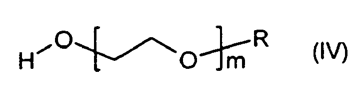 wobei
R und m die im Zusammenhang mit Formel (I) festgelegte Bedeutung haben.

Als Ether der Formel (IV) werden bevorzugt Ethylenglykolmonomethylether, Diethylenglykolmonomethylether oder Triethylenglykolmonomethylether eingesetzt.

Die Reaktanden werden typischerweise im Molverhältnis 1:10 bis 4:1 (Maleinsäureester zu Alkohol der Formel (IV)) bei Temperaturen von 100 bis 200°C, vorzugsweise von 120 bis 160°C in Gegenwart von Katalysatoren umgesetzt.

Als Katalysatoren werden typischerweise Verbindungen auf Basis von Zinn, Titan, Alkali- oder Erdalkalimetallen eingesetzt. Bevorzugte Katalysatoren sind zinnbasierte Verbindungen wie z.B.
Dibutylzinnoxid oder Dibutylzinndilaurat.

Der bei der Umesterung freiwerdende Alkohol wird zur Gleichgewichtsverschiebung bevorzugt destillativ entfernt. Der Fortschritt der Reaktion kann dabei an der Menge des abdestillierten Alkohols verfolgt werden. Die Reaktion wird typischerweise solange fortgesetzt, bis die theoretisch berechnete Menge an Alkohol abdestilliert ist.

Für den Fall, dass der Ether der Formel (IV) bezogen auf die Summe der vorliegenden Esterfunktionen in überschüssiger Menge eingesetzt wurde, kann dieser ebenfalls nach Abschluss der Umesterung abdestilliert werden.

Die so erhaltenen Maleinsäureester der Formel (II) werden dann vorzugsweise ohne weiteren Reinigungsschritt in die Umsetzung zum Polyasparaginsäureester eingesetzt. Eine destillative Aufreinigung kann grundsätzlich erfolgen, sollte aber nur bei solchen Produkten durchgeführt werden, die Molgewichte von unter 300 g/mol aufweisen, da ansonsten bei der Destillation Produktzersetzung auftreten kann.

Ein anderer Weg, die Maleinsäureester der Formel (II) herzustellen, ist die Umsetzung von Maleinsäureanhydrid mit Ethern der Formel (IV), wobei bei Temperaturen von 80 bis 160°C gearbeitet wird und das Reaktionswasser durch geeignete Destillationsverfahren entfernt wird. Zusätzlich werden saure Katalysatoren wie Schwefelsäure, p-Toluolsulfonsäure oder saure Ionenaustauscher mit verwendet.

Dieser Weg findet insbesondere dann Anwendung, wenn Ethylenglykolmonoalkylether als Ether der Formel (IV) eingesetzt wird.

Die erfindungsgemäßen hydrophilen Polyasparaginsäureester lassen sich problemlos in Wasser lösen bzw. zu stabilen Dispersionen formulieren. Dazu reicht gewöhnlich ein Vermischen des Esters mit Wasser, wobei, falls gewünscht auch mechanische Hilfsmittel wie Hochgeschwindigkeitsrührer oder Dispergatoren eingesetzt werden können.

Solche Dispersionen bzw. Lösungen haben typischerweise Konzentrationen bezogen auf den Polyasparaginsäureester von 30 bis 95 Gew.-%, bevorzugt 50 bis 90 Gew.-%.

Daher sind ein weiterer Gegenstand der Erfindung wässrige Lösungen oder Dispersionen enthaltend die erfindungsgemäßen Polyasparaginsäureester.

Ebenfalls Gegenstand der vorliegenden Erfindung sind 2K-Beschichtungsmittel zur Herstellung von Polyurethan-Polyharnstoff-Beschichtungen, enthaltend
a) mindestens einen erfindungsgemäßen hydrophilen Polyasparaginsäureester der Formel (I),
b) Wasser,
c) mindestens ein Polyisocyanat und
d) gegebenenfalls Hilfs- und Zusatzstoffe.

Das NCO : NH-Äquivalentverhältnis beträgt dabei typischerweise 0,5 : 1 bis 3,0 : 1, bevorzugt 0,8 : 1 bis 2,5 : 1.

Geeignete Polyisocyanate der Komponente c) sind organische Polyisocyanate mit einer mittleren NCO-Funktionalität von mindestens 2 und einem Molekulargewicht von mindestens 140 g/mol. Gut geeignet sind vor allem (i) unmodifizierte organische Polyisocyanate des zahlenmittleren Molekulargewichtsbereichs von 140 bis 300 g/mol, (ii) Lackpolyisocyanate eines zahlenmittleren Molekulargewichts von 300 bis 1000 g/mol sowie (iii) Urethangruppen-aufweisende NCO-Prepolymere mit zahlenmittleren Molekulargewichten > 1000 g/mol oder Gemische aus (i) bis (iii).

Beispiele für Polyisocyanate der Gruppe (i) sind 1,4-Diisocyanatobutan, 1,6-Diisocyanatohexan (HDI), 1,5-Diisocyanato-2,2-dimethylpentan, 2,2,4- bzw. 2,4,4-Trimethyl-1,6-diisocyanatohexan, 1-Isocyanato-3,3,5-trimethyl-5-isocyanatomethyl-cyclohexan (IPDI), 1-Isocyanato-1-methyl-4-(3)-isocyanatomethyl-cyclohexan, Bis-(4-isocyanatocyclohexyl)methan, 1,10-Diisocyanatodecan, 1,12-Diisocyanato-dodecan, Cyclohexan-1,3- und -1,4-diisocyanat, Xylylendiisocyanat-Isomere, Triisocyanatononan (TIN), 2,4-Diisocyanatotoluol oder dessen Gemische mit 2,6-Diisocyanatotoluol mit bevorzugt, bezogen auf Gemische, bis zu 35 Gew.-% 2,6-Diisocyanatotoluol, 2,2'-, 2,4'-, 4,4'-Diisocyanatodiphenylmethan oder technische Polyisocyanatgemische der Diphenylmethanreihe oder beliebige Gemische der genannten Isocyanate.

Polyisocyanate der Gruppe (ii) sind die an sich bekannten Lackpolyisocyanate. Unter dem Begriff "Lackpolyisocyanate" sind im Rahmen der Erfindung Verbindungen oder Gemische von Verbindungen zu verstehen, die durch an sich bekannte Oligomerisierungsreaktion von einfachen Diisocyanaten der unter (i) beispielhaft genannten Art erhalten werden. Geeignete Oligomerisierungsreaktionen sind z.B. die Carbodiimidisierung, Dimerisierung, Trimerisierung, Biuretisierung, Harnstoffbildung, Urethanisierung, Allophanatisierung und/oder Cyclisierung unter Ausbildung von Oxadiazinstrukturen. Oftmals laufen bei der "Oligomerisierung" mehrere der genannten Reaktionen gleichzeitig oder nacheinander ab.

Bevorzugt handelt es sich bei den "Lackpolyisocyanaten" (ii) um Biuretpolyisocyanate, Isocyanuratgrupen-aufweisende Polyisocyanate, Isocyanurat- und Uretdiongruppen-aufweisende Polyisocyanatgemische, Urethan- und/oder Allophanatgruppen-aufweisende Polyisocyanate oder um Isocyanurat- und Allophanatgruppen-aufweisende Polyisocyanatgemische auf Basis einfacher Diisocyanate.

Die Herstellung von derartigen Lackpolyisocyanaten ist bekannt und beispielsweise in der DE-A 1 595 273, DE-A 3 700 209 und DE-A 3 900 053 oder in der EP-A-0 330 966, EP-A 0 259 233, EP-A- 0 377 177, EP-A-0 496 208, EP-A-0 524 501 oder US-A 4 385 171 beschrieben.

Polyisocyanate der Gruppe (iii) sind die an sich bekannten Isocyanatgruppen aufweisenden Prepolymere auf Basis von einfachen Diisocyanaten der oben beispielhaft genannten Art und/oder auf Basis von Lackpolyisocyanaten (ii) einerseits und organischen Polyhydroxyverbindungen eines über 300 g/mol liegenden zahlenmittleren Molekulargewichts andererseits. Während es sich bei den Urethangruppen-aufweisenden Lackpolyisocyanaten der Gruppe (ii) um Derivate von niedermolekularen Polyolen des zahlenmittleren Molekulargewichtsbereichs von 62 bis 300 g/mol handelt, geeignete Polyole sind beispielsweise Ethylenglykol, Propylenglykol, Trimethylolpropan, Glycerin oder Gemische dieser Alkohole, werden zur Herstellung der NCO-Prepolymeren der Gruppe (iii) Polyhydroxylverbindungen mit zahlenmittleren Molekulargewichten von über 300 g/mol, bevorzugt über 500 g/mol, besonders bevorzugt von 500 bis 8000 g/mol eingesetzt. Derartige Polyhydroxylverbindungen sind insbesondere solche, die pro Molekül 2 bis 6, bevorzugt 2 bis 3 Hydroxylgruppen aufweisen und aus der Gruppe, bestehend aus Ether-, Ester-, Thioether-, Carbonat- und Polyacrylatpolyolen und Gemischen aus derartigen Polyolen ausgewählt sind.

Bei der Herstellung der NCO-Prepolymeren (iii) können die genannten höhermolekularen Polyole auch in Abmischungen mit den genannten niedermolekularen Polyolen zur Anwendung gelangen, so dass unmittelbar Gemische aus niedermolekularen, Urethangruppen aufweisenden Lackpolyisocyanaten (ii) und höhermolekularen NCO-Prepolymeren (iii) resultieren, die ebenfalls als erfindungsgemäße Ausgangskomponente (C) geeignet sind.

Zur Herstellung der NCO-Prepolymeren (iii) oder deren Gemische mit den Lackpolyisocyanaten (ii) werden Diisocyanate (i) der oben beispielhaft genannten Art oder Lackpolyisocynatate der unter (ii) beispielhaft genannten Art mit den höhermolekularen Hydroxylverbindungen oder deren Gemischen mit niedermolekularen Polyhydroxylverbindungen der beispielhaft genannten Art unter Einhaltung eines NCO/OH Äquivalentverhältnisses von 1,1:1 bis 40:1, bevorzugt 2:1 bis 25:1 unter Urethanbildung umgesetzt. Gegebenenfalls kann bei Verwendung eines Überschusses an destillierbarem Ausgangsdiisocyanat dieser im Anschluss an die Umsetzung destillativ entfernt werden, so dass monomerenfreie NCO-Prepolymere, d.h. Gemische aus Ausgangsdiisocyanaten (i) und echten NCO-Prepolymeren (iii) vorliegen, die ebenfalls als Komponente (A) eingesetzt werden können.

Niedrigviskose, hydrophilierte Polyisocyanate mit freien Isocyanatgruppen auf Basis aliphatischer, cycloaliphatischer, araliphatischer und/oder aromatischer Isocyanate, besonders bevorzugt aliphatischer oder cycloaliphatischer Isocyanate können auch eingesetzt werden.

Eine Hydrophilierung der Polyisocyanate ist z.B. durch Umsetzung mit unterschüssigen Mengen an einwertigen, hydrophilen Polyetheralkoholen möglich. Die Herstellung derartiger hydrophilierter Polyisocyanate ist beispielsweise in der EP-A 0 540 985, S. 3, Z 55 - S. 4 Z. 5 beschrieben. Gut geeignet sind auch die in der EP-A-959087, S. 3 Z. 39 - 51 beschriebenen Allophanatgruppen enthaltenden Polyisocyanate, die durch Umsetzung monomerenarmer Polyisocyanate mit Polyethylenoxidpolyetheralkoholen unter Allophanatisierungsbedingungen hergestellt werden. Auch die in der DE-A 100 078 21, S. 2 Z. 66 - S. 3 Z. 5, beschriebenen wasserdispergierbaren Polyisocyanatgemische auf Basis von Triisocyanatononan sind geeignet, sowie mit ionischen Gruppen (Sulfonat-, Phosphonatgruppen) hydrophilierte Polyisocyanate, wie sie z.B. in der DE 10024624, S. 3 Z. 13 - 33 beschrieben sind. Ebenso möglich ist die Hydrophilierung durch Zusatz handelsüblicher Emulgatoren.

Prinzipiell ist natürlich auch der Einsatz von Mischungen verschiedener Polyisocyanate der vorstehend genannten Art möglich.

Ggf. in Komponente d) enthaltene Hilfs- und Zusatzstoffe sind z. B. oberflächenaktive Substanzen, Schleifwachse, interne Trennmittel, Füllstoffe, Farbstoffe, Pigmente, Flammschutzmittel, Hydrolyseschutzmittel, Mikrobizide, Verlaufshilfsmittel, Antioxidantien wie 2,6-Di-tert-butyl-4-methylphenol, UV-Absorber vom Typ 2-Hydroxyphenyl-benzotriazol oder Lichtschutzmittel vom Typ der am Stickstoffatom substituierten oder unsubstituierten HALS-Verbindungen wie Tinuvin® 292 und Tinuvin® 770 DF (Ciba Spezialitäten GmbH, Lampertheim, DE) oder andere handelsübliche Stabilisierungsmittel, wie sie beispielsweise in "Lichtschutzmittel für Lacke" (A. Valet, Vincentz Verlag, Hannover, 1996) und "Stabilization of Polymeric Materials" (H. Zweifel, Springer Verlag, Berlin, 1997, Appendix 3, S. 181-213) beschrieben sind, oder beliebige Gemische dieser Verbindungen.

Typischerweise werden bei der Herstellung der erfindungsgemäßen Beschichtungsmittel die Einzelkomponenten a), b) und gegebenenfalls d) miteinander vermischt. Anschließend wird Komponente c), gegebenenfalls gemischt mit weiteren Bestandteilen von d), zugegeben und vermischt.

Beschichtungsmittel basierend auf den erfindungsgemäßen wässrigen Polyasparaginsäureestern können nach an sich bekannten Methoden wie beispielsweise durch Spritzen, Streichen, Fluten oder mit Hilfe von Walzen oder Rakeln auf beliebige Substrate aufgetragen werden. Als Substrate eignen sich beispielsweise Metall, Holz, Glas, Stein, keramische Materialien, Beton, harte und flexible Kunststoffe, Textilien, Leder oder Papier.

Die Aushärtung kann bei Raumtemperatur oder bei erhöhten Temperatur ausgeführt werden.

### Beispiele

Sofern nicht abweichend vermerkt, beziehen sich alle Prozentangaben auf das Gewicht.

Die Pendelhärten wurden nach König bestimmt DIN 53157.

Die dynamischen Viskositäten wurden bei 23°C mit einem Rotationsviskosimeter (ViscoTester® 550, Thermo Haake GmbH, D-76227 Karlsruhe) bestimmt.

Die OH-Zahl wurde nach DIN 53240 T.2 bestimmt.

### Polyisocyanat 1

Bayhydur® 3100 (Handelsprodukt der Bayer MaterialScience AG, Leverkusen, DE), hydrophiles aliphatisches Polyisocyanat auf Basis von 1,6 Hexandiisocyanat mit einem NCO-Gehalt von 17,4 Gew.-% und einer Viskosität bei 23°C von 2800 mPa·s.

### Polyisocyanat 2

Desmodur® N3400 (Handelsprodukt der Bayer MaterialScience AG, Leverkusen, DE), aliphatisches Polyisocyanat auf Basis von 1,6-Hexandiisocyanat mit einem NCO-Gehalt von 21,8 Gew.-% und einer Viskosität bei 23°C von 150 mPa·s.

### Hydrophober Polyasparaginsäureester

Desmophen® NH1420 (Handelsprodukt der Bayer MaterialScience AG, Leverkusen, DE), Polyasparaginsäureester auf Basis von 4,4'-Diamino-dicyclohexylmethan und Maleinsäurediethylester.

### Herstellung des hydrophilen Maleinsäureesters

432 g Maleinsäuredimethylester, 493 g Triethylenglykolmonomethylether und 0,9 g Dibutylzinnoxid wurden in einen Dreihalskolben mit Rührer, Destillationskolonne, Thermometer eingewogen und auf 140°C aufgeheizt. Parallel zur einsetzenden Umesterungsreaktion wurde das freigesetzte Methanol unter Vakuum (30 mbar) abdestilliert. Nachdem die theoretische Menge von 96 g Methanol überdestilliert war, wurde die Umsetzung unterbrochen. Das resultierende Produkt hatte eine OH-Zahl von 0.

### Herstellung des hydrophilen Polyasparaginsäureesters

131 g 4,4'-Diamino-dicyclohexylmethan wurden in einem Dreihalskolben mit Rührer, Destillationskolonne, Thermometer und Stickstoffdurchleitung vorgelegt und auf 50°C aufgeheizt. Anschließend wurden 345 g des vorstehend hergestellten Maleinsäureesters so zugetropft, dass die Temperatur 50°C nicht überschritt. Nach beendeter Zugabe wurde die Temperatur auf 60°C erhöht und für 24 Stunden nachgerührt. Das Produkt hatte eine Viskosität bei 23°C von 1390 mPa·s.

Durch Zugabe von Wasser konnten wässrige Lösungen dieses Polyasparaginsäureesters hergestellt werden. Eine 50 Gew.-%ige Lösung in Wasser hatte eine Viskosität bei 23°C von 25 mPa.s und eine 80 Gew.-%ige Lösung hatte eine Viskosität bei 23°C von 270 mPa·s.

### Lackherstellung

Der hydrophile Polyasparaginsäureester wurde gemäß nachstehender Tabelle in Wasser gelöst und mit der angegebenen Menge des Polyisocyanats unter Rühren versetzt. Anschließend wurde diese Bindemittelmischung mit einem Aufziehramen von 180 µm auf einer Glassplatte appliziert und 24 Stunden bei Raumtemperatur ausgehärtet. Im Falle der erfindungsgemäßen hydrophilen Polyasparaginsäureesters wurden klare, elastische Filme erhalten. Da der vergleichsweise eingesetzte hydrophobe Polyasparaginsäureester nicht in Wasser löslich war, konnte keine homogene wässrige Lösung erhalten werden. Dies führte aufgrund von Phasenseparation der Komponenten zu unbrauchbaren Filmen.

**Tabelle 1: Zusammensetzung und Eigenschaften der Lacke (Mengenangaben in Gewichtsteilen)**

| **Beispiel** | **1** | **2** | **3** | **4** | **4** |
|---|---|---|---|---|---|
| Hydrophiler Polyasparaginsäureester | 80 | 80 | 50 | 80 | 80 |
| | | | | | |
| Hydrophober Polyasparaginsäureester | - | - | - | - | - |
| | | | | | |
| Wasser | 20 | 20 | 50 | 20 | 20 |
| | | | | | |
| Polyisocyanat 1 | 50 | 100 | 63 | 40 | 80 |
| | | | | | |
| Polyisocyanat 2 | - | - | - | - | - |
| | | | | | |
| Aussehen Film | klar | klar | klar | klar | klar |
| | | | | | |
| Pendelhärte nach 7 Tagen | 14 s | 31 s | 21 s | 10 s | 13 s |

## Patentansprüche

1. Hydrophile Polyasparaginsäureester der allgemeinen Formel (I), 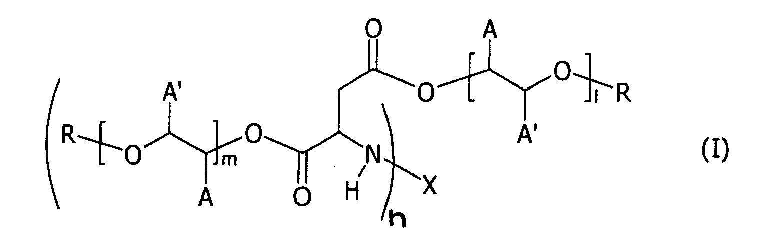 wobei
R ein C₁-C₄-Alkylrest ist,
A, A' unabhängig voneinander Wasserstoff oder Methyl sind,
X ein n-valenter aliphatischer, araliphatischer oder cycloaliphatischer Rest mit 1 bis 20 C-Atomen ist, der gegebenenfalls Etherbrücken enthält,
l, m natürliche Zahlen von 0 bis 10 sind deren Summe 1 + m = 1 bis 20 beträgt sowie
n eine natürliche Zahl von 1 bis 3 ist.

2. Hydrophile Polyasparaginsäureester gemäß Anspruch 1, **dadurch gekennzeichnet, dass** diese der Formel (I) entsprechen, wobei A und A' Wasserstoff, X ein n-valenter, gegebenenfalls ethergruppenhaltiger aliphatischer oder cycloaliphatischer Rest mit 1 bis 10 C-Atomen, 1 und m unabhängig voneinander 1, 2 oder 3 sind.

3. Hydrophile Polyasparaginsäureester gemäß Anspruch 1 oder 2, **dadurch gekennzeichnet, dass** sie zahlenmittlere Molekulargewichte von 300 bis 1000 g/mol aufweisen.

4. Hydrophile Polyasparaginsäureester gemäß einem der Ansprüche 1 bis 3, **dadurch gekennzeichnet, dass** sie bei 23°C Viskositäten von 50 bis 5000 mPas aufweisen.

5. Wässrige Lösungen oder Dispersionen enthaltend hydrophile Polyasparaginsäureester gemäß einem der Ansprüche 1 - 4.

6. Wässrige Lösungen oder Dispersionen gemäß Anspruch 5, **dadurch gekennzeichnet, dass** sie Gehalte bezogen auf den Polyasparaginsäureester von 30 bis 95 Gew.-% aufweisen.

7. Verfahren zur Herstellung hydrophiler Polyasparaginsäureester gemäß einem der Ansprüche 1 bis 4, bei dem Maleinsäureester der allgemeinen Formel (II), 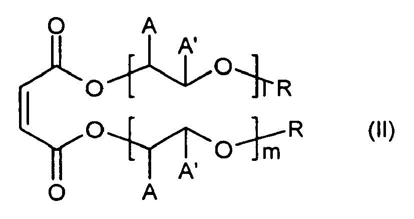 wobei
R ein C₁-C₄-Alkylrest ist,
A, A' unabhängig voneinander Wasserstoff oder Methyl sind,
l, m unabhängig voneinander natürliche Zahlen von 0 bis 10 sind und deren Summe 1 + m = 1 bis 20 beträgt
mit einem n-valenten Amin der Formel (III), 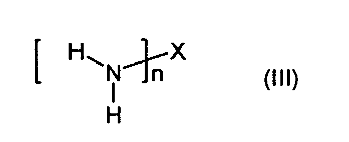 wobei
X ein n-valenter aliphatischer, araliphatischer oder cycloaliphatischer Rest mit 1 bis 20 C-Atomen ist, der gegebenenfalls Etherbrücken enthält,
n eine natürliche Zahlen von 1 bis 3 ist,
bei Temperaturen von 0 bis 100°C, vorzugsweise von 15 bis 50°C bevorzugt lösemittelfrei umgesetzt wird.

8. 2-Komponenten-Beschichtungsmittel enthaltend
a) mindestens einen hydrophilen Polyasparaginsäureester gemäß einem der Ansprüche 1 bis 4,
b) Wasser,
c) mindestens ein Polyisocyanat und
d) gegebenenfalls Hilfs- und Zusatzstoffe.

9. Beschichtungen erhältlich unter Verwendung von Polyasparaginsäureestern gemäß einem der Ansprüche 1 bis 4.

10. Substrate beschichtet mit Beschichtungen gemäß Anspruch 9.

## Claims

1. Hydrophilic polyaspartic esters of the general formula (I) 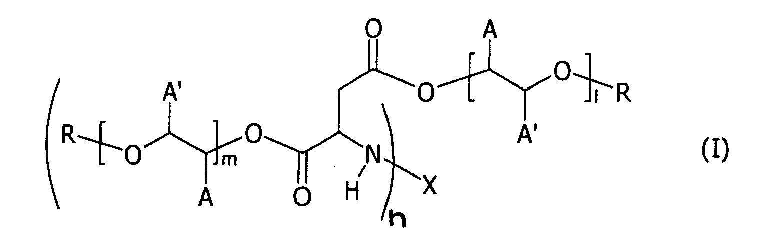 where
R is C₁-C₄ alkyl radical,
A and A' independently of one another are hydrogen or methyl,
X is an n-valent aliphatic, araliphatic or cycloaliphatic radical having 1 to 20 carbon atoms and optionally containing ether bridges,
l and m are natural numbers from 0 to 10 whose sum 1 + m = 1 to 20, and
n is a natural number from 1 to 3.

2. Hydrophilic polyaspartic esters according to Claim 1, **characterized in that** they are of the formula (I) in which A and A' are hydrogen, X is an n-valent aliphatic or cycloaliphatic radical having 1 to 10 carbon atoms and optionally containing ether groups, and 1 and m independently of one another are 1, 2 or 3.

3. Hydrophilic polyaspartic esters according to Claim 1 or 2, **characterized in that** they have number-average molecular weights of 300 to 1000 g/mol.

4. Hydrophilic polyaspartic esters according to any one of Claims 1 to 3, **characterized in that** they have viscosities at 23°C of 50 to 5000 mPas.

5. Aqueous solutions or dispersions comprising hydrophilic polyaspartic esters according to any one of Claims 1 - 4.

6. Aqueous solutions or dispersions according to Claim 5, **characterized in that** their polyaspartic ester contents are from 30% to 95% by weight.

7. Process for preparing hydrophilic polyaspartic esters according to any one of Claims 1 to 4 by reacting maleic esters of the general formula (II) 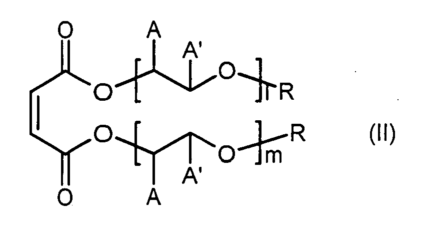 where
R is a C₁-C₄ alkyl radical,
A and A' independently of one another are hydrogen or methyl, and
l and m independently of one another are natural numbers from 0 to 10 and their sum 1 + m = 1 to 20
with an n-valent amine of the formula (III) 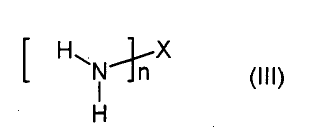 where
X is an n-valent aliphatic, araliphatic or cycloaliphatic radical having 1 to 20 carbon atoms and optionally containing ether bridges and
n is a natural number from 1 to 3
at temperatures of 0 to 100°C, preferably of 15 to 50°C, preferably without solvent.

8. 2-Component coating composition comprising
a) at least one hydrophilic polyaspartic ester according to any one of Claims 1 to 4,
b) water,
c) at least one polyisocyanate and
d) optionally auxiliaries and additives.

9. Coatings obtainable using polyaspartic esters according to any one of Claims 1 to 4.

10. Substrates coated with coatings according to Claim 9.

## Revendications

1. Esters polyaspartiques hydrophiles de formule générale (I), 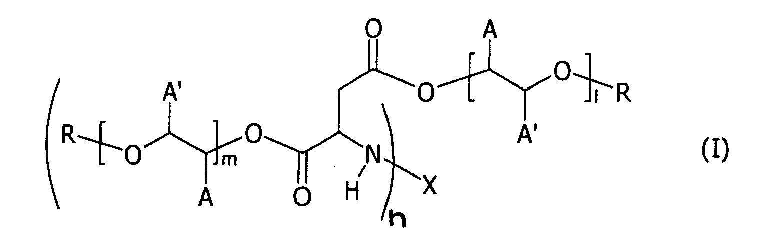 dans laquelle
R représente un groupe alkyle en C₁-C₄,
A et A' représentent chacun, indépendamment l'un de l'autre, l'hydrogène ou un groupe méthyle,
X représente un radical aliphatique, araliphatique ou cycloaliphatique en C₁-C₂₀, de valence n, qui peut contenir le cas échéant des ponts éther,
l et m sont des nombres naturels allant de 0 à 10 et dont la somme l + m = 1 à 20 et
n est un nombre naturel allant de 1 à 3.

2. Esters polyaspartiques hydrophiles selon la revendication 1, **caractérisés en ce qu'**ils répondent à la formule (I) dans laquelle A et A' représentent l'hydrogène, X un radical aliphatique ou cycloaliphatique en C₁-C₁₀, de valence n, contenant le cas échéant des groupes éther, et 1 et m sont égaux chacun, indépendamment l'un de l'autre, à 1, 2 ou 3.

3. Esters polyaspartiques hydrophiles selon la revendication 1 ou 2, **caractérisés en ce qu'**ils ont des poids moléculaires moyens, moyenne en nombre, de 300 à 1000 g/mol.

4. Esters polyaspartiques hydrophiles selon l'une des revendications 1 à 3, **caractérisés en ce qu'**ils ont des viscosités de 50 à 5000 mPa·s à 23°C.

5. Solutions ou dispersions aqueuses contenant des esters polyaspartiques hydrophiles selon l'une des revendications 1 à 4.

6. Solutions ou dispersions aqueuses selon la revendication 5, **caractérisées en ce que** leur teneur en ester polyaspartique est de 30 à 95 % en poids.

7. Procédé pour la préparation des esters polyaspartiques hydrophiles selon l'une des revendications 1 à 4, selon lequel on fait réagir à des températures de 0 à 100°C, de préférence de 15 à 50°C, de préférence en l'absence de solvants, des esters maléiques de formule générale (II), 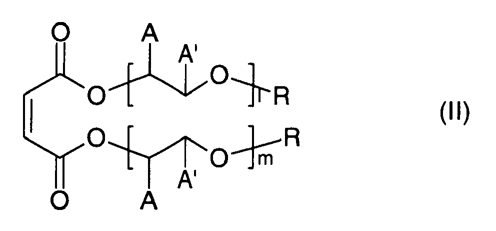 dans laquelle
R représente un groupe alkyle en C₁-C₄,
A et A' représentent chacun, indépendamment l'un de l'autre, l'hydrogène ou un groupe méthyle,
l et m sont chacun, indépendamment l'un de l'autre, des nombres naturels de 0 à 10 dont la somme l + m = 1 à 20,
avec une amine de valence n répondant à la formule (III) 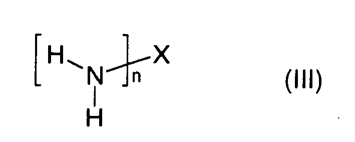 dans laquelle
X représente un radical aliphatique, araliphatique ou cycloaliphatique en C₁-C₂₀, de valence n, contenant le cas échéant des ponts éther,
n est un nombre naturel allant de 1 à 3.

8. Produit de revêtement à deux composants contenant
a) au moins un ester polyaspartique hydrophile selon l'une des revendications 1 à 4,
b de l'eau,
c) au moins un polyisocyanate et
d) le cas échéant des produits auxiliaires et additifs.

9. Revêtements obtenus à l'aide des esters polyaspartiques selon l'une des revendications 1 à 4.

10. Supports portant des revêtements selon la revendication 9.
